Europäisches Patentamt

(19) European Patent Office

Office européen des brevets

(11) EP 0 930 285 A1

(12) DEMANDE DE BREVET EUROPEEN

(43) Date de publication:
21.07.1999 Bulletin 1999/29

(51) Int. Cl.$^6$: C07C 11/08

(21) Numéro de dépôt: 98403303.5

(22) Date de dépôt: 24.12.1998

(84) Etats contractants désignés:
AT BE CH CY DE DK ES FI FR GB GR IE IT LI LU
MC NL PT SE
Etats d'extension désignés:
AL LT LV MK RO SI

(30) Priorité: 14.01.1998 FR 9800397

(71) Demandeur:
INSTITUT FRANCAIS DU PETROLE
92500 Rueil Malmaison (FR)

(72) Inventeurs:
• Dorbon, Michel
  75004 Paris (FR)
• Hugues, François
  Charly, 69390 Vernaison (FR)
• Viltard, Jean-Charles
  38200 Vienne (FR)
• Didillon, Blaise
  92500 Rueil Malmaison (FR)
• Forestiere, Alain
  69390 Vernaison (FR)

(54) Procédé pour l'obtention de 1-butène

(57) On décrit un procédé pour l'obtention de 1-butène à partir de 2-butènes dans lequel :

- une charge contenant au moins l'un des isomères du 2-butène est introduite par la ligne 1 dans une zone de distillation C ;
- l'effluent de fond de colonne est évacué de la zone de distillation par la ligne 2 ;
- une partie du flux qui circule dans la ligne 2 est évacuée du dispositif par la ligne 7 ; l'autre partie du flux qui circule dans la ligne 2 étant réchauffée au moyen d'un échangeur de chaleur avant d'alimenter la ligne 4 d'adduction de la charge dans la zone d'hydro-isomérisation R ;
- la ligne 4 est aussi alimentée par la ligne 3 en un flux contenant au moins de l'hydrogène ;
- l'effluent d'hydro-isomérisation est évacué de la zone d'hydro-isomérisation R par la ligne 5, refroidi par un échangeur de chaleur, puis introduit à nouveau dans la zone de distillation C ;
- et l'effluent de tête de colonne de la zone de distillation est évacué par la ligne 6.

FIG.1

**Description**

La présente invention concerne le domaine de la production de 1-butène. Le 1-butène est un composé important pour la synthèse de copolymères, par exemple avec de l'éthylène pour les polyéthylènes linéaires basse densité, ou pour la synthèse d'oxyde de butène.

L'étude de l'art antérieur a fait ressortir deux documents qui décrivent des procédés dans lesquels au moins un des produits isolés est du 1-butène, ce sont les brevets FR-B-2 527 201 et EP-B-0 129 900. Le brevet FR-B-2 527 201 décrit un procédé en plusieurs étapes à partir d'une charge d'hydrocarbures à 4 atomes de carbone. Après avoir subi plusieurs traitements dont une réaction d'éthérification avec un alcool aliphatique, puis plusieurs traitements de séparation des différents constituants du mélange, le 1-butène est récupéré en fraction de tête d'une colonne de distillation, la fraction de queue étant constituée des autres composés et de 1-butène non récupéré. Cette fraction de queue est ensuite envoyée dans une zone d'isomérisation, où elle est partiellement transformée en isobutène. Le brevet EP-B-0 129 900 décrit un procédé pour produire du 1-butène par isomérisation à partir d'un mélange d'hydrocarbures contenant des 2-butènes. Ce document soulève un problème important qui est la durée de vie du catalyseur d'isomérisation et donc de la régénération de ce catalyseur. Le type de catalyseur utilisé est un catalyseur acide et les conditions opératoires de la réaction requièrent une température de réaction de l'ordre de 250°C à 400°C. D'autre part ce procédé génère de l'isobutène, qu'il est difficile de séparer du 1-butène, même si ce composé n'est présent qu'à l'état de traces.

La présente invention concerne un procédé en plusieurs étapes pour l'obtention de 1-butène à partir d'une charge comprenant des hydrocarbures à 4 atomes de carbone par molécule dont au moins l'un des isomères du 2-butène (le 2-butène se présente sous les formes cis et trans), ce procédé comprenant au moins une étape de distillation et au moins une étape d'hydro-isomérisation, ces étapes pouvant en outre être confondues.

Parmi les avantages de la présente invention, on notera la possibilité d'obtenir du 1-butène avec des rendements supérieurs à 100 % en masse par rapport au 1-butène présent dans la charge. Par rapport aux procédés d'isomérisation, on notera que l'utilisation d'un catalyseur d'hydro-isomérisation permet de travailler à des températures significativement plus faibles. Cette température d'hydro-isomérisation moins élevée fait que le taux de craquage n'est pas détectable. La formation de coke, qui est le principal responsable de la désactivation du catalyseur, est également très faible.

Le catalyseur utilisé est très sélectif de la réaction d'isomérisation par rapport aux réactions d'hydrogénation. De plus, la quantité d'hydrogène nécessaire pour la réaction d'hydro-isomérisation est très faible. Les pertes dues à l'hydrogénation des butènes sont donc très faibles et il n'est pas indispensable d'ajouter un moyen pour séparer l'hydrogène de l'effluent de sortie du réacteur d'hydro-isomérisation. On n'exclut cependant pas du cadre de la présente invention les réalisations dans lesquelles on utilise un moyen pour isoler l'hydrogène lorsque l'effluent de sortie du réacteur d'hydro-isomérisation contient de l'hydrogène.

L'invention concerne un procédé pour l'obtention de 1-butène dans lequel une charge contenant au moins l'un des isomères du 2-butène est envoyée dans une zone de distillation associée à une zone d'hydro-isomérisation.

L'invention concerne aussi un dispositif pour la mise en oeuvre du procédé selon l'invention.

La charge traitée par le procédé selon l'invention peut être une coupe contenant des hydrocarbures à 4 atomes de carbone, dont au moins l'un des isomères du 2-butène. Elle peut provenir par exemple d'un vapocraquage, d'un craquage catalytique en lit fuide (FCC) ou encore d'une isomérisation squelettale.

La charge contient généralement du butadiène, des butanes (n-butane et isobutane), des butènes (isobutène, 1-butène et 2-butènes, formes cis et trans). Cette charge peut être introduite dans la zone de distillation du procédé selon l'invention sans traitement préalable. Cependant, certaines utilisations requièrent du 1-butène de haute pureté. Pour celles-ci, il est donc nécessaire de séparer le 1-butène des produits dont le point d'ébullition est proche ou inférieur au point d'ébullition du 1-butène. Ces produits sont généralement le butadiène, l'isobutane et l'isobutène.

Pour éliminer le butadiène du mélange, on lui fait généralement subir une hydrogénation sélective, l'effluent de sortie du réacteur d'hydrogénation sélective ne contient alors plus que des butanes (n-butane et isobutane), des butènes (isobutène, 1-butène et 2-butènes, formes cis et trans), ainsi que des traces de butadiène.

On élimine habituellement l'isobutène par extraction ou en l'utilisant comme réactif d'une réaction. L'isobutène peut par exemple être oligomérisé, par exemple selon un procédé dit "Polynaphta" développé par la demanderesse., Il peut aussi réagir avec certains alcools selon des réactions d'éthérification pour donner par exemple du méthyl tertio butyl éther (MTBE) ou de l'éthyl tertio butyl éther (ETBE). L'isobutane est généralement éliminé par distillation.

Les traitements d'élimination du butadiène et de l'isobutène peuvent être effectués sur la charge avant son introduction dans la zone de distillation du procédé selon l'invention ou sur l'effluent de tête de colonne du procédé selon l'invention. De préférence, ils seront effectués avant l'introduction de la charge dans la zone de distillation. L'isobutane est généralement éliminé dans l'effluent de tête du procédé selon l'invention.

L'invention concerne un procédé pour l'obtention de 1-butène dans lequel une charge contenant au moins l'un des isomères du 2-butène est envoyée dans une zone de distillation associée à une zone d'hydro-isomérisation. La charge de la zone de distillation est introduite à au moins un niveau de la colonne de distillation, la charge de la zone d'hydro-isomérisation est prélevée à la hauteur d'au moins un niveau de la zone de distillation et elle représente au moins une partie du liquide coulant dans la zone de distillation. Ce niveau de la zone de distillation peut aussi être le fond de la colonne de distillation. L'effluent de la zone d'hydro-isomérisation est au moins en partie réintroduit dans la zone de distillation à au moins un niveau de réintroduction. Suivant le procédé selon l'invention, la fraction de tête de l'effluent de la zone de distillation contient au moins du 1-butène.

La zone de distillation comprend généralement au moins une colonne munie d'au moins un interne de distillation choisi dans le groupe formé par les plateaux simples, les plateaux à multi-déversoirs, les garnissages en vrac et les garnissages structurés, ainsi qu'il est connu de l'homme du métier, par exemple de telle sorte que l'efficacité globale totale est au moins égale à cinq étages théoriques. Dans les cas connus de l'homme du métier où la mise en oeuvre d'une seule colonne pose des problèmes, on préfère généralement scinder ladite zone de façon à utiliser finalement au moins deux colonnes qui, mises bout à bout, réalisent ladite zone, c'est-à-dire que la zone de rectification, la zone réactionnelle éventuelle et la zone d'épuisement se répartissent sur les colonnes.

La zone de distillation est associée à une zone d'hydro-isomérisation dans laquelle au moins une partie des 2-butènes présents dans la charge sont convertis en 1-butène. Cette zone d'hydro-isomérisation peut être externe à la zone de distillation ou incorporée à la zone de distillation, elle peut aussi être en partie externe à la zone de distillation et en partie incorporée à la zone de distillation.

La zone d'hydro-isomérisation du procédé selon l'invention peut être définie comme une zone où une réaction est réalisée en présence d'un catalyseur d'hydro-isomérisation et d'un flux gazeux comprenant de l'hydrogène.

La zone d'hydro-isomérisation comprend généralement au moins un lit catalytique d'hydro-isomérisation contenant un catalyseur d'hydro-isomérisation. De préférence, la zone réactionnelle comprend de 1 à 6 lits catalytiques. La zone d'hydro-isomérisation réalise au moins partiellement l'hydro-isomérisation des 2-butènes en 1-butène.

La zone réactionnelle d'hydro-isomérisation peut être au moins en partie externe à la zone de distillation. Elle comprend alors au moins un réacteur, de préférence un seul réacteur. Selon cette réalisation, le procédé selon l'invention permet d'isomériser des 2-butènes en 1-butène à l'extérieur de la zone de distillation, éventuellement dans des conditions de pression et/ou de température différentes de celles utilisées dans la colonne.

La charge de la zone de distillation peut être introduite à n'importe quel niveau de la colonne de distillation, par exemple à plusieurs niveaux d'introduction, mais de préférence à un seul niveau de la colonne de distillation. On ne sortirait pas du cadre de la présente invention en introduisant dans la zone de distillation plusieurs charges de compositions différentes à plusieurs niveaux d'introduction.

A partir d'une charge comprenant des hydrocarbures à 4 atomes de carbone, dont du butabiène, des butanes (n-butane et isobutane), des butènes (isobutène, 1-butène et 2-butènes formes cis et trans), on récupère, en tête de colonne de distillation, principalement le butadiène, l'isobutane, l'isobutène et le 1-butène, en fond de colonne de distillation, on récupère principalement le n-butane et les 2-butènes.

Chaque palier de la colonne peut être défini par un couple pression-température et à chaque palier correspond une composition du mélange des différents constituants. Ainsi, les mélanges riches en 1-butène se situent dans les paliers supérieurs de la colonne (haut de la colonne), les mélanges riches en 2-butènes se trouvent dans les paliers inférieurs de la colonne (bas de la colonne).

La charge de la zone d'hydro-isomérisation est prélevée à au moins un niveau de prélèvement de la zone de distillation, de préférence à un seul niveau de celle-ci. Le prélèvement de la colonne de distillation est généralement effectué à un niveau où la valeur du rapport de la quantité des 2-butènes sur la quantité de 1-butène est supérieure à sa valeur à l'équilibre thermodynamique à l'entrée de la zone de réaction. Le prélèvement de la colonne de distillation est généralement effectué à un niveau situé sous le niveau d'introduction de la charge dans ladite colonne de distillation, plus particulièrement, le prélèvement peut être effectué en fond de colonne de distillation.

Le prélèvement de la colonne de distillation effectué en fond de colonne de distillation peut n'être qu'en partie introduit dans la zone d'hydro-isomérisation. Habituellement, on prévoit aussi l'évacuation d'une autre partie de cette fraction. D'autre part, lorsque la zone d'hydro-isomérisation est entièrement externe à la zone de distillation, le prélèvement de la zone de distillation peut subir un traitement avant d'être introduit dans la zone d'hydro-isomérisation. Ce traitement peut par exemple être une séparation des différents constituants du mélange, une purification de l'effluent, une réaction autre qu'une hydro-isomérisation.

La réaction d'hydro-isomérisation est la suivante :

$$1\text{-butène} \rightarrow 2\text{-butènes (cis et trans)}$$

Outre la réaction d'hydro-isomérisation notée ci-dessus, des réactions secondaires d'hydrogénation qui produisent essentiellement des butanes en petites quantités ont lieu, ces réactions contribuant à diminuer le rendement en butène-1. Cependant, la quantité d'hydrogène nécessaire à l'hydro-isomérisation étant très faible, on veillera à ne pas introduire un excès trop important d'hydrogène, de plus, on choisira un catalyseur très sélectif en faveur de l'isomérisation.

La réaction d'hydro-isomérisation est effectuée en présence d'hydrogène avec un catalyseur supporté comprenant généralement au moins un métal choisi dans le groupe formé par le nickel et les métaux nobles du groupe VIII tel que le ruthénium, le rhodium, le palladium, l'osmium, l'iridium et le platine. Ce catalyseur peut être utilisé tel quel ou de préférence déposé sur un support.

On emploie le plus souvent un catalyseur supporté comprenant du palladium. La teneur en métal noble du catalyseur est habituellement d'environ 0,01 à 2 % en masse.

Ce catalyseur est habituellement traité par un composé contenant du soufre, puis par de l'hydrogène avant son utilisation en zone d'hydro-isomérisation. Le métal se trouve avantageusement sous forme réduite, au moins pour 50 % de sa masse totale.

Dans le cas de l'utilisation de nickel, la proportion de nickel par rapport à la masse totale du catalyseur est d'environ 5 à 70 %, et de façon préférée d'environ 10 à 70 %. De plus, on utilise généralement un catalyseur tel que la taille moyenne des cristallites de nickel est inférieure à 10 nm, de préférence inférieure à 8 nm, de façon encore plus préférée inférieure à 6 nm. Mais tout autre catalyseur d'hydro-isomérisation connu de l'homme du métier peut également être choisi. Le catalyseur est généralement sulfuré *in situ ou ex situ,* de telle façon que du soufre soit chimisorbé sur une partie au moins du métal.

Le support est généralement choisi dans le groupe formé par l'alumine, les silices-alumines, la silice, les zéolithes, le charbon actif, les argiles, les ciments alumineux, les oxydes de terres rares et les oxydes alcalino-terreux, seuls ou en mélanges. On utilise de préférence un support à base d'alumine ou de silice, de surface spécifique d'environ 1 à 300 $m^2$/g, de préférence d'environ 5 à 100 $m^2$/g.

A titre d'exemples non limitatifs de catalyseurs utilisables dans le cadre de la présente invention, on peut citer les catalyseurs commerciaux tels que celui vendu par la société Catalysts and Chemicals sous la référence C-31, celui vendu par la société Girdler Corporation sous la référence G-55 ou, de préférence, ceux vendus par la société Procatalyse sous les références LD-265, LD-265S, LD-267 et LD-267R.

La réaction d'hydro-isomérisation est effectuée en présence d'hydrogène, l'hydrogène pouvant provenir de toutes sources produisant de l'hydrogène à au moins 50 % en volume de pureté, de préférence au moins 80 % en volume de pureté et de façon encore plus préférée au moins 90% en volume de pureté.

Lorsque la zone d'hydro-isomérisation est au moins en partie incorporée à la zone de distillation, les conditions opératoires de ladite partie incorporée sont liées aux conditions opératoires de la distillation. Elle est alors réalisée sous une pression absolue d'environ 1 et 50 bar, de préférence d'environ 2 et 30 bar et de manière plus préférée d'environ 4 à 15 bar (1 bar = $10^5$ Pa), avec un taux de reflux d'environ 1 à 70, et de préférence d'environ 5 à 40. La température de tête de zone de distillation est d'environ 0 à 200 °C et la température de fond de zone de distillation est généralement d'environ 5 à 250 °C. La réaction d'hydro-isomérisation est conduite dans des conditions qui sont le plus généralement intermédiaires entre celles établies en tête et en fond de zone de distillation, à une température d'environ 0 à 250 °C, et de préférence d'environ 50 à 200 °C. Le liquide soumis à l'hydro-isomérisation est alimenté par un flux gazeux comprenant, de préférence en majeure partie, de l'hydrogène.

Dans la partie externe de la zone d'hydro-isomérisation, le catalyseur est disposé dans tout lit catalytique suivant toute technologie connue de l'homme du métier dans des conditions opératoires (température, pression etc.) indépendantes ou non, de préférence indépendantes, des conditions opératoires de la zone de distillation.

Dans la partie de la zone d'hydro-isomérisation externe à la zone de distillation, les conditions opératoires sont généralement les suivantes. La pression absolue requise pour cette étape d'hydro-isomérisation est généralement d'environ 1 à 70 bar, de préférence d'environ 2 à 30 bar. La température opératoire de la zone d'hydro-isomérisation est généralement d'environ 0 à 250 °C, de préférence d'environ 50 à 200 °C. La vitesse spatiale (définie comme le volume de charge par le volume de catalyseur et par heure et notée VVH) au sein de ladite zone d'hydro-isomérisation, calculée par rapport au catalyseur, est généralement d'environ 0,1 à 150 $h^{-1}$ et plus particulièrement d'environ 4 à 50 $h^{-1}$ et de façon encore plus préférée d'environ 10 à 30 $h^{-1}$. Le débit d'hydrogène correspondant est tel que le rapport molaire $H_2$/hydrocarbures entrant dans la zone d'hydro-isomérisation est de préférence au moins égal à $10^{-5}$. Ce rapport est le plus souvent d'environ $10^{-5}$ à environ 3 et très fréquemment d'environ $10^{-4}$ à environ 1.

Dans les descriptions qui suivent, il n'est pas fait mention du détail des appareillages bien connus de l'homme du métier, comme par exemple les rebouilleurs et ballons de reflux des colonnes de distillation. Dans les figures suivantes, les lignes correspondant à l'admission des fractions de reflux dans la colonne sont notées 60 et les lignes correspondant à l'admission des fractions rebouillies dans la colonne sont notées 70.

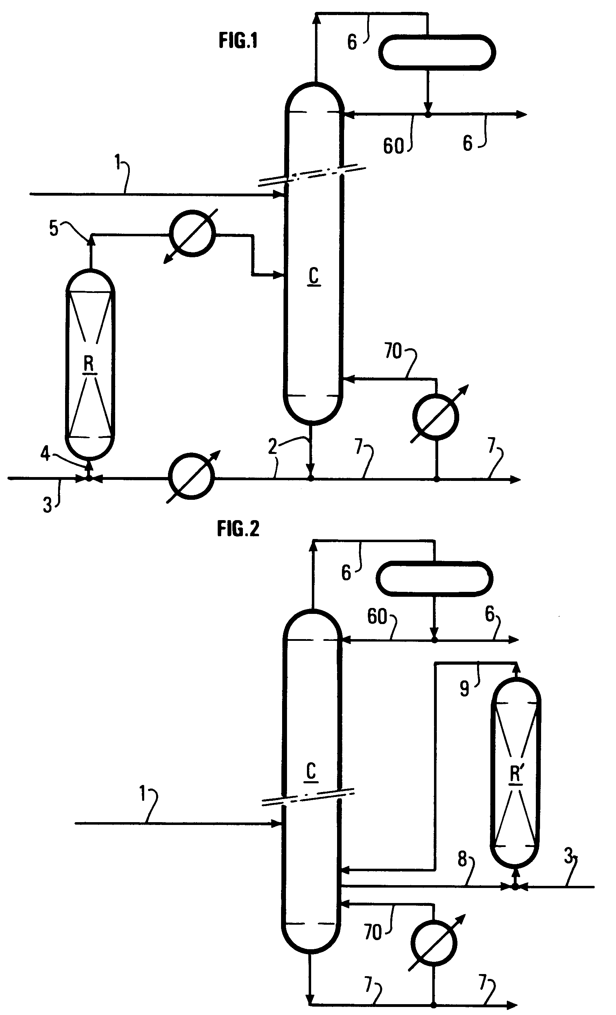

La figure 1 représente une réalisation simple du procédé selon l'invention.

La charge à traiter est introduite par la ligne 1 dans une zone de distillation C. L'effluent de fond de colonne est évacué de la zone de distillation par la ligne 2. Une partie du flux qui circule dans la ligne 2 est évacuée du dispositif par la ligne 7. L'autre partie du flux qui circule dans la ligne 2 est réchauffée au moyen d'un échangeur de chaleur avant d'alimenter la ligne 4 d'adduction de la charge dans la zone d'hydro-isomérisation R. La ligne 4 est aussi alimentée via la ligne 3 par un flux contenant au moins de l'hydrogène. L'effluent d'hydro-isomérisation est évacué de la zone d'hydro-isomérisation R par la ligne 5, il est refroidi par un échangeur de chaleur, puis introduit à nouveau dans la zone de distillation C. L'effluent de tête de colonne de la zone de distillation est évacué par la ligne 6.

La figure 2 représente une autre réalisation simple du procédé selon l'invention.

Selon la figure 2, la charge à traiter est introduite par la ligne 1 dans une zone de distillation C. Par la ligne 8, un prélèvement est effectué à un niveau intermédiaire de la colonne de distillation C. La ligne 8 alimente la zone d'hydro-isomérisation R', cette zone d'hydro-isomérisation R' étant aussi alimentée par un flux contenant de l'hydrogène via la ligne 3. L'effluent d'hydro-isomérisation est évacué de la zone d'hydro-isomérisation R' et réintroduite dans la zone de distillation C par la ligne 9. L'effluent de fond de colonne est évacué de la zone de distillation C par la ligne 7. L'effluent de tête de colonne de la zone de distillation C est évacué du dispositif par la ligne 6.

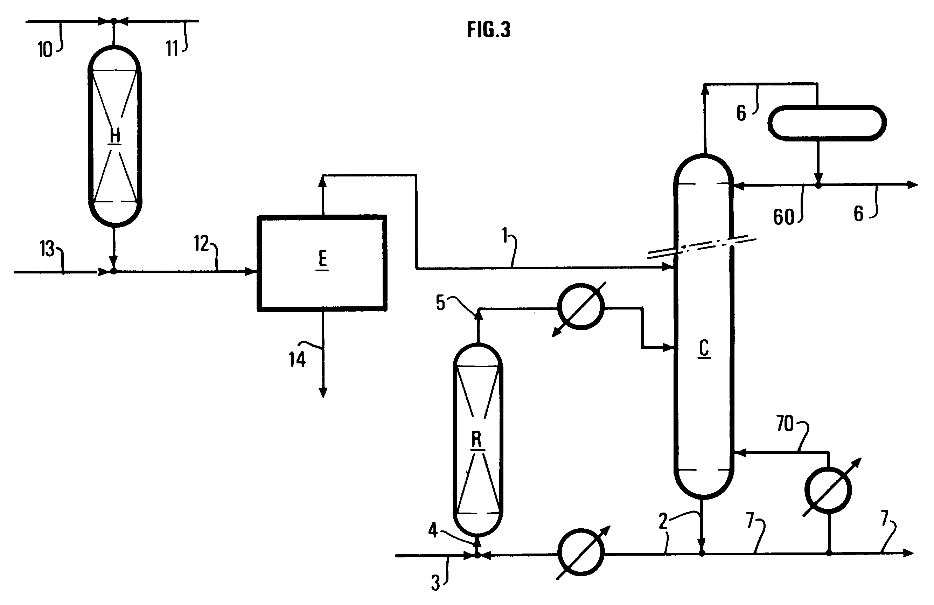

La figure 3 représente une réalisation dans laquelle la charge de la zone de distillation subit au préalable plusieurs réactions.

La charge à traiter est introduite par la ligne 10 dans une zone d'hydrogénation sélective H, cette zone d'hydrogénation sélective H étant alimentée en hydrogène par la ligne 11. L'effluent de la zone H est évacué par la ligne 12, la ligne 13 alimente la ligne 12 en une charge contenant un alcool. La charge est envoyée dans la zone d'étherification associée à une zone de séparation E par la ligne 12. Le produit de la réaction d'éthérification E est évacué par la ligne 14 et les composés qui n'ont pas réagi sont envoyés par la ligne 1 dans la zone de distillation C. L'effluent de fond de colonne est évacué de la zone de distillation par la ligne 2. Une partie du flux qui circule dans la ligne 2 est évacuée du dispositif par la ligne 7. L'autre partie du flux qui circule dans la ligne 2 est réchauffée au moyen d'un échangeur de chaleur avant d'alimenter la ligne 4 d'adduction de la charge dans la zone d'hydro-isomérisation R. La ligne 4 est aussi alimentée par la ligne 3 en un flux contenant au moins de l'hydrogène. L'effluent d'hydro-isomérisation est évacué de la zone d'hydro-isomérisation R par la ligne 5, il est refroidi par un échangeur de chaleur, puis il est introduit à nouveau dans la zone de distillation C. L'effluent de tête de colonne de la zone de distillation est évacué par la ligne 6.

Les exemples suivants ilustrent l'invention sans en limiter la portée.

## Exemples

Dans le cadre de la demande de brevet PCT WO 96/02 086, des tests ont été effectués afin de décrire une cinétique de transformation du 1-butène en 2-butènes dans le cas où le rapport de la quantité de 1-butène sur la quantité de 2-butènes est supérieur à la valeur qu'il prend dans les conditions de l'équilibre thermodynamique. Le catalyseur utilisé pour effectuer cette cinétique est le catalyseur d'hydro-isomérisation LD-267R commercialisé par la société Procatalyse.

Les résultats de cette cinétique ont été utilisés pour la réaction de transformation des 2-butènes en 1-butène dans le cas où le rapport de la quantité des 2-butènes sur la quantité de 1-butène est supérieur à la valeur qu'il prend dans les conditions de l'équilibre thermodynamique. Ces résultats ont permis de simuler le procédé selon l'invention au moyen de logiciels adaptés. Le logiciel utilisé pour cette simulation est commercialisé sous le nom de Pro 2[®] par la société SIMCI. Le modèle choisi pour simuler les exemples qui suivent correspond à une réalisation conforme à la figure 1 de la présente description.

Dans les exemples ci-dessous les abréviations suivantes sont employées : < $C_4$ pour hydrocarbures contenant strictement moins de 4 atomes de carbone, i $C_4$ pour isobutane, i $C_4^=$ pour isobutène, $C_4^=$ 1 pour 1-butène, n $C_4$ pour n-butane, $C_4^=$ 2 tr pour 2-trans butène, $C_4^=$ 2 cis pour 2-cis butène.

## Exemple 1

L'unité comprend une zone de distillation et une zone d'hydro-isomérisation. La zone d'hydro-isomérisation est externe à la colonne de distillation, la colonne de distillation comprend 130 plateaux théoriques, numérotés de haut en bas. L'alimentation est effectuée au niveau du plateau n°80, le réacteur d'hydro-isomérisation est alimenté par un soutirage situé au niveau du plateau n°130 de la colonne de distillation et l'effluent d'hydro-isomérisation est réinjecté au niveau du plateau n°100. Le réacteur contient 7 tonnes de catalyseur.

Les conditions opératoires sont les suivantes : un taux de reflux de la colonne (reflux/distillat) de 17, une pression absolue en tête de colonne de 6,2 bar, une pression absolue en fond de colonne de 7 bar, une température de l'alimentation de la colonne : 61,6 °C, une température en tête de colonne de 51,7 °C, une température en fond de colonne de 64,6 °C, une température du réacteur de 75 °C, une pression absolue du réacteur de 7 bar, un débit total (comprenant le débit de la charge et le débit d'hydrogène) dans le réacteur d'hydro-isomérisation de 250 kmole/h.

Avec cette configuration et dans ces conditions opératoires, la simulation a conduit aux résultats figurant dans le tableau suivant.

| Constituants | Alimentation colonne (kmole/h) | Tête de colonne (kmole/h) | Fond de colonne (kmole/h) |
|---|---|---|---|
| < $C_4$ | 0,8 | 0,8 | 0 |
| i $C_4$ | 12,3 | 12,4 | 0 |
| i $C_4^=$ | 2,3 | 2,2 | 0 |
| $C_4^=$ 1 | 80,2 | 84,3 | 1,2 |
| n $C_4$ | 109,9 | 27,0 | 83,2 |
| $C_4^=$ 2 tr | 100,2 | 6,0 | 88,6 |
| $C_4^=$ 2 cis | 54,7 | 0 | 54,7 |
| $H_2$ | 0,4 | 0 | 0 |
| Total | 360,8 | 132,7 | 227,7 |

Ce tableau permet de calculer le rendement en 1-butène en tête de colonne : 105,1 % en masse, et le rendement de l'hydrogénation c'est-à-dire le rapport des butanes dans les effluents (tête et fond de colonne) / butanes dans l'alimentation qui est de 100,32 % en masse. On note aussi que la quantité d'hydrogène dans les effluents de tête et de fond de colonne est nulle.

## Exemple 2

L'unité comprend une zone de distillation et une zone d'hydro-isomérisation. La zone d'hydro-isomérisation est externe à la colonne de distillation, la colonne de distillation comprend 130 plateaux théoriques, numérotés de haut en bas. L'alimentation est effectuée au niveau du plateau n°80, le réacteur d'hydro-isomérisation est alimenté par un soutirage situé au niveau du plateau n°130 de la colonne de distillation et l'effluent d'hydro-isomérisation est réinjecté au niveau du plateau n°100. Le réacteur contient 7 tonnes de catalyseur.

Les conditions opératoires sont les suivantes : un taux de reflux de la colonne (reflux/distillat) de 30, une pression absolue en tête de colonne de 6,2 bar, une pression absolue en fond de colonne de 7 bar, une température de l'alimentation de la colonne : 62,5 °C, une température en tête de colonne de 53,4 °C, une température en fond de colonne de 65,8 °C, une température du réacteur d'hydro-isomérisation de 75 °C, une pression absolue du réacteur de 10 bar, un débit dans le réacteur (comprenant le débit de le charge et le débit d'hydrogène) de 2000 kmole/h.

Avec cette configuration et dans ces conditions opératoires, la simulation a conduit aux résultats figurant dans le tableau suivant.

| Constituants | Alimentation colonne (kmole/h) | Tête de colonne (kmole/h) | Fond de colonne (kmole/h) |
|---|---|---|---|
| < $C_4$ | 0,8 | 0,8 | 0 |
| i $C_4$ | 12,3 | 12,4 | 0 |
| i $C_4^=$ | 2,3 | 2,2 | 0 |
| $C_4^=$ 1 | 80,2 | 134,9 | 0,5 |
| n $C_4$ | 109,9 | 88,8 | 21,9 |
| $C_4^=$ 2 tr | 100,2 | 23,6 | 21,4 |
| $C_4^=$ 2 cis | 54,7 | 0,1 | 53,8 |
| $H_2$ | 0,9 | 0 | 0 |
| Total | 360,8 | 263,4 | 98 |

Ce tableau permet de calculer le rendement en 1-butène en tête de colonne : 168,2 % en masse, et le rendement de l'hydrogénation c'est-à-dire le rapport des butanes dans les effluents (tête et fond de colonne) / butanes dans l'alimentation qui est de 100,7 % en masse.

On note aussi que la quantité d'hydrogène dans les effluents de tête et de fond de colonne est nulle.

Ces exemples montrent que même lorsqu'on travaille à une température relativement basse, on obtient déjà de bons rendements en 1-butène, avec un faible rendement en produits d'hydrogénation.

**Revendications**

1.  Procédé pour l'obtention de 1-butène à partir de 2-butènes caractérisé en ce qu'une charge contenant au moins l'un des isomères du 2-butène est envoyée dans une zone de distillation associée à une zone d'hydro-isomérisation, la charge de la zone de distillation étant introduite à au moins un niveau de ladite zone de distillation, la charge de la zone d'hydro-isomérisation étant prélevée à la hauteur d'au moins un niveau de prélèvement de la zone de distillation et représentant au moins une partie du liquide coulant dans la zone de distillation, l'effluent de la zone d'hydro-isomérisation étant au moins en partie réintroduit dans la zone de distillation à au moins un niveau de réintroduction, de façon à sortir en tête de la zone de distillation un effluent contenant au moins du 1-butène.

2.  Procédé pour l'obtention de 1-butène à partir de 2-butènes selon la revendication 1 caractérisé en ce que la réaction d'hydro-isomérisation est effectuée dans au moins une zone externe à la zone de distillation.

3.  Procédé pour l'obtention de 1-butène à partir de 2-butènes selon la revendication 1 caractérisé en ce que la réaction d'hydro-isomérisation est effectuée dans au moins une zone incorporée à la zone de distillation.

4.  Procédé pour l'obtention de 1-butène à partir de 2-butènes selon la revendication 1 caractérisé en ce que la réaction d'hydro-isomérisation est effectuée en partie dans au moins une zone incorporée à la zone de distillation et en partie dans au moins une zone externe à la zone de distillation.

5.  Procédé pour l'obtention de 1-butène à partir de 2--butènes selon l'une des revendications 1 à 4 caractérisé en ce que la distillation est effectuée à une pression absolue d'environ 1 à 50 bar, avec un taux de reflux d'environ 1 à 70, avec une température de tête de zone de distillation d'environ 0 à 200 °C et une température de fond de zone de distillation d'environ 5 à 25 °C.

6.  Procédé pour l'obtention de 1-butène à partir de 2-butènes selon l'une des revendications 1 à 5 caractérisé en ce que l'hydro-isomérisation effectuée dans une zone incorporée à la zone de distillation est effectuée à une température d'environ 0 à 250 °C.

7.  Procédé pour l'obtention de 1-butène à partir de 2-butènes selon l'une des revendications 1 à 5 caractérisé en ce que l'hydro-isomérisation effectuée dans une zone externe à la zone de distillation est effectuée à pression absolue d'environ 1 à 70 bar, à une température d'environ 0 à 250 °C.

8.  Procédé pour l'obtention de 1-butène à partir de 2-butènes selon l'une des revendications 1 à 7 caractérisé en ce que l'on utilise dans la zone d'hydro-isomérisation un catalyseur supporté contenant au moins un métal choisi dans le groupe formé par le nickel et les métaux nobles du groupe VIII.

9.  Procédé pour l'obtention de 1-butène à partir de 2-butènes selon l'une des revendications 1 à 8 caractérisé en ce qu'il comprend une étape d'élimination de l'isobutène, cette étape étant effectuée avant ou après les étapes associées de distillation et d'hydro-isomérisation.

10. Procédé pour l'obtention de 1-butène à partir de 2-butènes selon l'une des revendications 1 à 9 caractérisé en ce qu'il comprend une étape d'élimination de l'isobutane, cette étape étant effectuée avant ou après les étapes associées de distillation et d'hydro-isomérisation

11. Procédé pour l'obtention de 1-butène à partir de 2-butènes selon l'une des revendications 1 à 10 caractérisé en ce qu'il comprend une étape d'élimination du butadiène, cette étape étant effectuée avant ou après les étapes associées de distillation et d'hydro-isomérisation.

12. Dispositif pour la mise en oeuvre du procédé selon l'une des revendications 1 à 8 caractérisé en ce qu'il comprend une zone de distillation associée à une zone d'hydro-isomérisation.

13. Dispositif selon la revendication 12 ou pour la mise en oeuvre du procédé selon la revendication 9 caractérisé en ce qu'il comprend une zone d'élimination de l'isobutène.

14. Dispositif selon la revendication 12 ou 13 ou pour la mise en oeuvre du procédé selon la revendication 10 caractérisé en ce qu'il comprend une zone d'élimination de l'isobutane.

15. Dispositif selon l'une des revendications 12 à 14 ou pour la mise en oeuvre du procédé selon la revendication 11 caractérisé en ce qu'il comprend une zone d'élimination du butadiène.

**FIG.1**

**FIG.2**

FIG.3

**Office européen des brevets**

**RAPPORT DE RECHERCHE EUROPEENNE**

Numéro de la demande

EP 98 40 3303

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A,D | EP 0 129 900 A (BASF AG) 2 janvier 1985<br>* revendications *<br>--- | 1 | C07C11/08 |
| A | FR 2 528 033 A (EXXON RESEARCH AND ENGINEERING COMPANY) 9 décembre 1983<br>* revendications *<br>----- | 7-9 | |

**DOMAINES TECHNIQUES RECHERCHES (Int.Cl.6)**

C07C

Le présent rapport a été établi pour toutes les revendications

| Lieu de la recherche | Date d'achèvement de la recherche | Examinateur |
|---|---|---|
| LA HAYE | 8 avril 1999 | Van Geyt, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE**
**RELATIF A LA DEMANDE DE BREVET EUROPEEN NO.**                    EP 98 40 3303

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-04-1999

| Document brevet cité au rapport de recherche | Date de publication | Membre(s) de la famille de brevet(s) | | Date de publication |
|---|---|---|---|---|
| EP 0129900 A | 02-01-1985 | DE | 3323021 A | 03-01-1985 |
| | | AT | 28067 T | 15-07-1987 |
| | | CA | 1219882 A | 31-03-1987 |
| | | JP | 60019724 A | 31-01-1985 |
| FR 2528033 A | 09-12-1983 | BE | 896987 A | 07-12-1983 |
| | | GB | 2121431 A | 21-12-1983 |
| | | JP | 59005129 A | 12-01-1984 |
| | | US | 4435609 A | 06-03-1984 |

EPO FORM P0460

Pour tout renseignement concernant cette annexe : voir Journal Officiel de l'Office européen des brevets, No.12/82